# EUROPEAN PATENT SPECIFICATION

(11) **EP 2 110 149 B1**
(45) Date of publication and mention of the grant of the patent: **03.05.2017**
(21) Application number: 09004481.9
(22) Date of filing: 27.03.2009
(51) Int. Cl.: A61B 5/15, A61M 5/00, A61M 25/06, A61B 5/153, A61M 5/32

(54) **Winged needle assembly and frangible cover**
Flügelnadelanordnung und zerbrechliche Hülle
Assemblage d'aiguille à ailettes et couvercle fragile

(30) Priority: 28.03.2008 US 40206 P; 20.06.2008 US 74360 P
(43) Date of publication of application: 21.10.2009
(73) Proprietor: Fenwal, Inc., Lake Zurich, IL 60047 (US)
(72) Inventor: West, Richard L., Lake Villa, IL 60046 (US); Jones, Mark B., Libertyville, IL 60048 (US)
(74) Representative: Geary, Stephen

(56) References cited:
- EP-A- 1 221 303
- EP-A- 1 340 458
- EP-A- 1 665 986
- US-A- 5 279 581
- US-A1- 2004 092 872
- US-A1- 2009 187 153

## Description

### Field of the Disclosure

The present subject matter relates to a medical needle assembly of the type commonly used in blood collection or withdrawal systems. More particularly, the present disclosure relates to a needle cover for use in a medical needle assembly according to the foregoing recitation.

### Description of Related Art

Blood collection sets, in their most basic form, typically include a collection container for receiving blood from a donor and a tube that provides a flow path from the blood donor to the collection container, the tube terminating in a needle for withdrawal of blood from a donor or patient. The needle assembly typically includes a needle or cannula attached to a needle hub which allows for manipulation of the needle assembly by the phlebotomist, nurse, or other medical professional.

Such needles are commonly shielded with a removable needle cover. Shielding the needle protects the medical professional from inadvertent contact with the sharpened needle tip. Shielding the needle also protects the needle from damage during shipping and transport. Shielding also preserves the sterility of the needle prior to use and ideally maintains the integrity of a closed blood collection system by preferably providing a hermetic seal between the needle cover and needle assembly. The needle cover may also optionally provide assurance to the end user that the needle has not been tampered with.

Examples of needle assemblies including associated needle covers are provided in U.S. Patent Nos. 4,402,682 and 4,496,352, both of which are hereby incorporated herein by reference. These patents disclose a needle assembly including a cannula attached to a base and to donor tubing. The needle covers are made of a plastic material, which forms a thermal bond with the base to provide a tamper evident seal. The needle covers described in the above-referenced patents also include an internal plug within the bore of the needle cover and located at the distal end of the needle cover. The plug is made of a resilient material and includes a pocket for enveloping and protecting the distal needle tip.

Another example of a needle assembly and associated needle cover is disclosed in U.S. Patent No. 4,551,138, which is hereby incorporated herein by reference. The needle cover disclosed therein includes a hollow body made of a sterilizable plastic material. The needle cover includes a resilient "layer" made of a polymeric elastomer located near the proximal open end of the needle cover. The diameter of the cylindrical resilient layer at the proximal end of the cover has a diameter that is smaller than the diameter of the needle post engaged by the needle cover. The needle cover is placed over the needle and engages the post of the needle hub. According to U.S. Patent No. 4,551,138, a hermetic seal is formed between the needle cover and the hub or post portion of the needle assembly.

US 2004/092872 describes a fluid transfer device including a tube holder, a fluid container, a double ended needle with forward sharpened tip and rearward sharpened tip. The needle cover is having a hub adapted to be secured to a base and a pair of wings (Fig.14). This publication does not describe the hub having a projection aligned with the bevel indicator, extending proximally away from the bevel indicator, and configured to be received by the channel of a needle base when a hub portion is secured to the base.

EP1221303 describes a device having a circular cross-section and a two-piece structure with wings pivoting with pieces of the body.

### Summary

The invention provides a medical needle assembly as defined in claim 1.

There are several embodiments of the present subject matter which may be embodied in the devices and systems described and claimed below. These aspects may be employed alone or in combination with other aspects of the subject matter described herein.

Suitably, in one embodiment, a not according to the claimed invention, medical needle assembly comprises a cannula, a base secured to the cannula, and a needle cover. The needle cover comprises a hub portion adapted to be secured to the base. The hub portion includes a pair of outwardly extending wings and a raised orientation member, with the wings being foldable to overlay the raised orientation member and contact the raised orientation member and each other.

Suitably, in another embodiment, not according to the claimed invention, a medical needle assembly comprises a cannula, a base secured to the cannula, and a needle cover. The needle cover comprises a hub portion adapted to be secured to the base. The hub portion includes a bottom surface, a pair of sidewalls extending from the bottom surface, and a pair of guidewalls extending toward each other from upper ends of the sidewalls. The hub portion further includes a pair of wings extending outwardly from the sidewalls, with the wings being foldable to overlay at least a portion of the guidewalls and contact the guidewalls and each other.

Suitably, in yet another embodiment, not according to the claimed invention, a medical needle assembly comprises a cannula, a base secured to the cannula, a winged attachment piece, and a needle cover. The winged attachment piece comprises a ring portion and a pair of wings extending from the ring portion. The needle cover comprises a hub portion secured to the base.

The ring portion is rotatably received on a portion of the hub portion and an outwardly extending anti-rotation member of the hub portion cooperates with the ring portion to limit rotation of the winged attachment piece with respect to the hub portion.

### Brief Description of the Drawings

Fig. 1 is a plan view of a disposable blood collection kit;
Fig. 2A is a perspective view of a medical needle assembly incorporating a needle cover according to an aspect of the present disclosure;
Fig. 2B is a top plan view of the medical needle assembly of Fig. 2A;
Fig. 2C is a cross-sectional side view of the medical needle assembly of Fig. 2B, taken through the line 2C-2C;
Fig. 3 is an exploded view of another medical needle assembly incorporating a needle cover according to an aspect of the present disclosure;
Fig. 4A is a side view of a cannula and base of a medical needle assembly suitable for use with needle covers according to the present disclosure;
Fig. 4B is an end view of the cannula and base of Fig. 4A;
Fig. 5A is a top plan view of a needle cover according to an aspect of the present disclosure;
Fig. 5B is a cross-sectional side view of the needle cover of Fig. 5A;
Fig. 5C is a cross-sectional end view of the needle cover of Fig. 5A, taken through the line 5C-5C;
Fig. 5D is a cross-sectional end view of the needle cover of Fig. 5A, taken through the line 5D-5D;
Fig. 6 is a perspective view of another medical needle assembly incorporating a needle cover according to an aspect of the present invention;
Fig. 7 is a top plan view of the medical needle assembly of Fig. 6;
Fig. 8 is a bottom plan view of the medical needle assembly of Fig. 6;
Fig. 9 is a side elevational view of the medical needle assembly of Fig. 6;
Fig. 10 is a rear elevational view of the medical needle assembly of Fig. 6;
Fig. 11 is a front elevational view of the medical needle assembly of Fig. 6, with a cap portion of the needle cover removed and foldable wings pressed together for phlebotomization of a patient or donor;
Fig. 12 is a perspective view of a cannula and base employed by the medical needle assembly of Fig. 6;
Fig. 13 is an exploded view of an alternative embodiment of the medical needle assembly of Fig.6 not according to the claimed invention;
Fig. 14 is a side elevational view of a cannula and base employed by the medical needle assembly of Fig. 13;
Fig. 15 is a bottom elevational view of the cannula and base of Fig. 14;
Fig. 16 is a rear elevational view of the medical needle assembly of Fig. 13;
Fig. 17 is an exploded view of another medical needle assembly incorporating a needle cover according to an aspect of the present disclosure;
Fig. 18 is a perspective view of the medical needle assembly of Fig. 17 in an assembled condition;
Fig. 19 is a cross-sectional view of the medical needle assembly of Fig. 18, taken through the line 19-19 of Fig. 18;
Fig. 20 is a front elevational view of the medical needle assembly of Fig. 18, with a cap portion of the needle cover removed for phlebotomization of a patient or donor;
Fig. 21 is a rear elevational view of a medical needle assembly according to an aspect of the present disclosure, with a winged attachment piece thereof being in a detached condition;
Fig. 22 is a rear elevational view of the medical needle assembly of Fig. 21, with the winged attachment piece thereof being in an attached condition;
Fig. 23 is a front elevational view of a medical needle assembly according to an aspect of the disclosure, not according to the claimed invention, with a winged attachment piece thereof being in a non-shielding position;
Fig. 24 is a front elevational view of the medical needle assembly of Fig. 23, with a winged attachment piece thereof being in a shielding position;
Fig. 25 is a perspective view of a dual-bevel needle tip;
Fig. 26 is a perspective view of another medical needle assembly incorporating a needle cover according to an aspect of the present disclosure;
Fig. 27 is a perspective view of the medical needle assembly of Fig. 26, with a cap portion of the needle cover removed;
Fig. 28 is a perspective view of the medical needle assembly of Fig. 26, with foldable wings thereof pressed together for phlebotomization of a patient or donor;
Fig. 29 is a perspective view of the medical needle assembly of Fig. 26, with a cap portion of the needle cover removed and foldable wings thereof pressed together for phlebotomization of a patient or donor;
Fig. 30 is a front elevational view of a medical needle assembly according to an aspect of the present disclosure, with a winged attachment piece thereof being in a detached condition; and
Fig. 31 is a front elevational view of the medical needle assembly of Fig. 30, with the winged attachment piece thereof being in an attached condition.

### Description of the Illustrated Embodiments

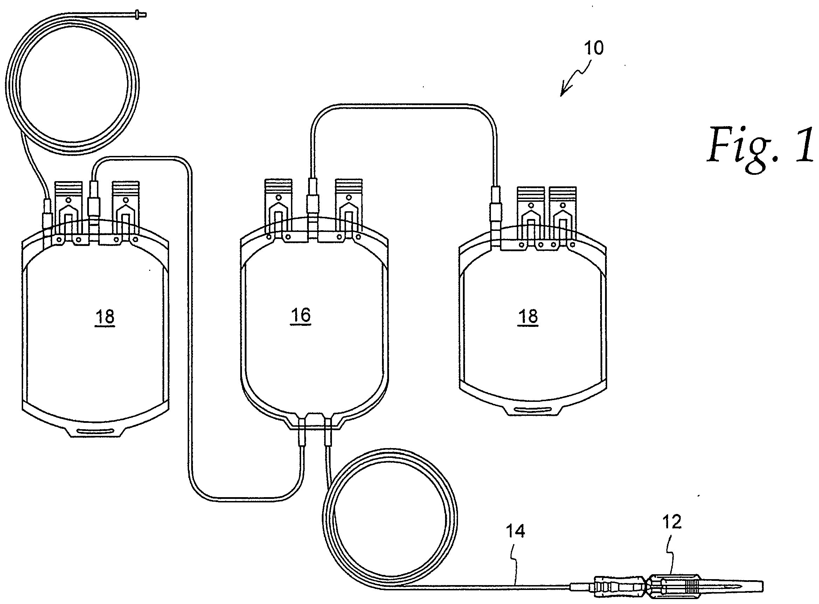

The embodiments disclosed herein are for the purpose of providing the required description of the present subject matter. These embodiments are only exemplary, and may be embodied in various forms. Therefore, specific details disclosed herein are not to be interpreted as limiting the subject matter as defined in the accompanying claims. Fig. 1 shows a disposable blood collection and processing kit of the type commonly used in whole blood donations. The disposable set 10 includes a medical needle assembly 12 attached to tubing 14, which provides a flow path to a collection container 16. The disposable set 10 may further include satellite containers 18 for receiving separated blood components and/or for holding a storage medium for a separated component, as will be recognized by those of skill in the art. The system shown in Fig. 1 is just one example of a blood collection processing set. Other examples are shown and described in, for example, U.S. Patent Application Publication No. 2005/0148993, which is hereby incorporated herein by reference.

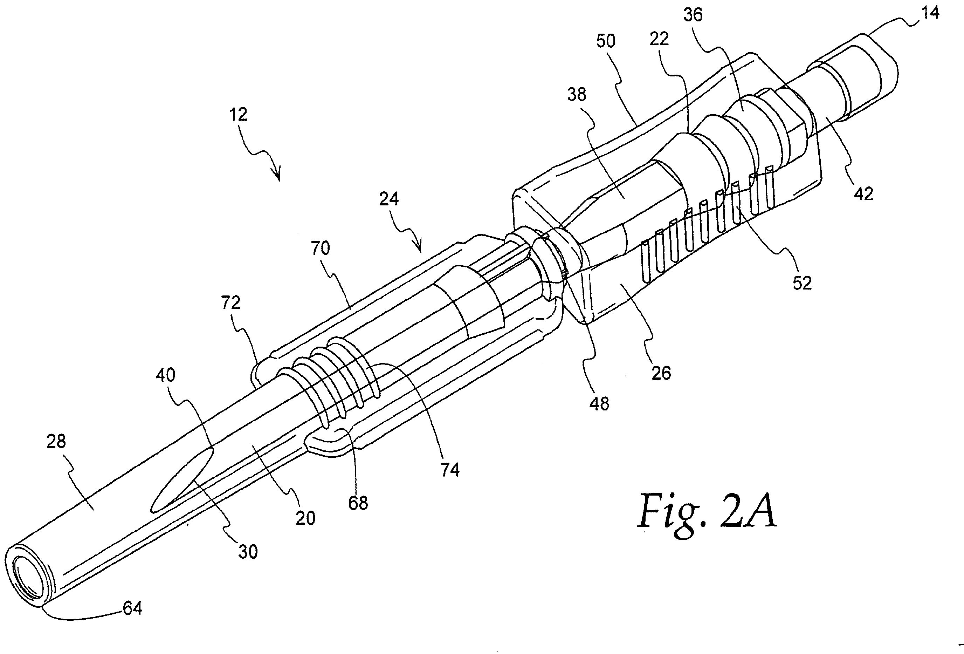

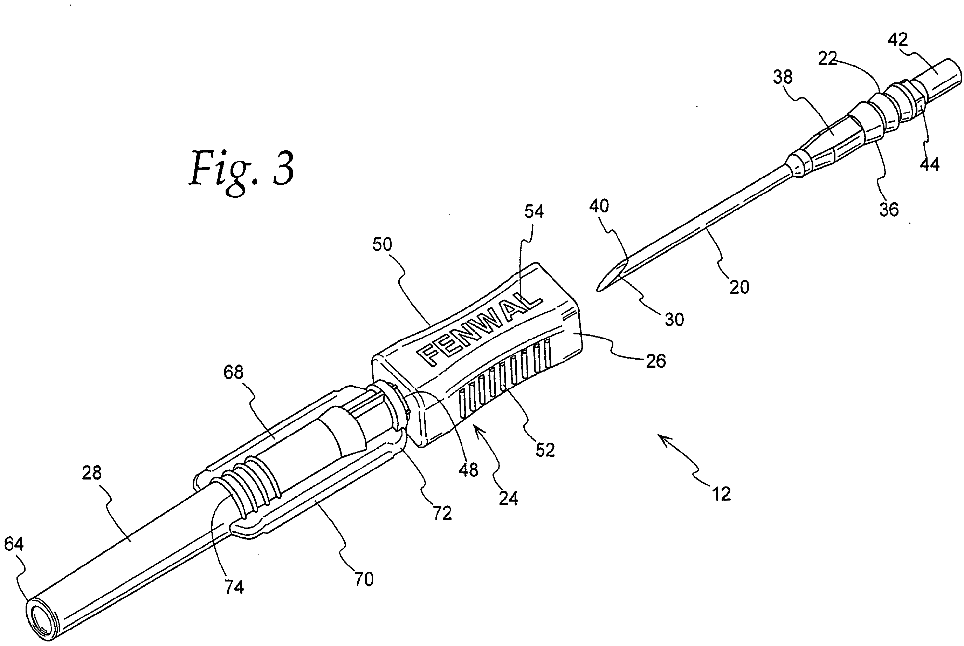

Figs. 2A-2C show a medical needle assembly 12 embodying various aspects of the present subject matter. An exploded view of a similar medical needle assembly 12 is shown in Fig. 3. The assembly 12 includes a cannula or needle 20, a base 22 secured to the cannula 20, and a needle cover 24. The cannula 20 is secured to the base 22, and the base 22 is received within a hub portion 26 of the needle cover 24, and a cap portion 28 of the needle cover 24 encloses at least a beveled distal end 30 of the cannula 20. The cap portion 28 is removed to allow access to the cannula 20 and subsequent phlebotomy of a donor or patient.

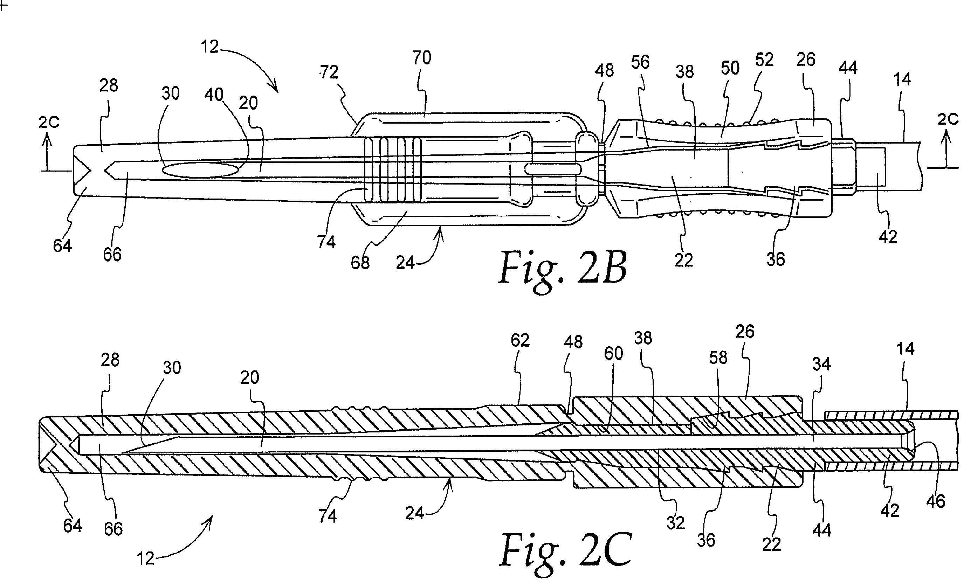

The cannula 20 (shown more particularly in Figs. 3-4B) is adapted for insertion into the vein of the blood donor. The cannula 20 is typically made of stainless steel or other suitable alloy metal. The cannula 20 may be further coated by one or more lubricants to facilitate venipuncture. Needle lubricants include, but are not limited to cross-linked, silicone-based lubricants and/or simple lubricating oils that will be known to those of ordinary skill in the art. As seen in Fig. 2C, the cannula 20 is hollow and terminates at its distal end in a sharpened and beveled tip 30.

The cannula 20 is attached to a post or base 22. The base 22 is a generally tubular member that includes a bore 32 (Fig. 2C) for receiving the proximal end or shank 34 of the cannula 30. In one embodiment, the base 22 may be attached to the cannula 20 by overmolding over the proximal end 34 of the cannula. In another embodiment, the proximal end 34 of the cannula 20 may be inserted into the hollow bore 32 of the base 22 and secured by adhesive bonding or other known techniques. Suitable adhesives include, without being limited to, those that are curable by ultraviolet (UV) radiation. Other ways of combining or otherwise attaching the cannula 20 and the base 22, such as by a press-fit, may also be employed without departing from the scope of the present description.

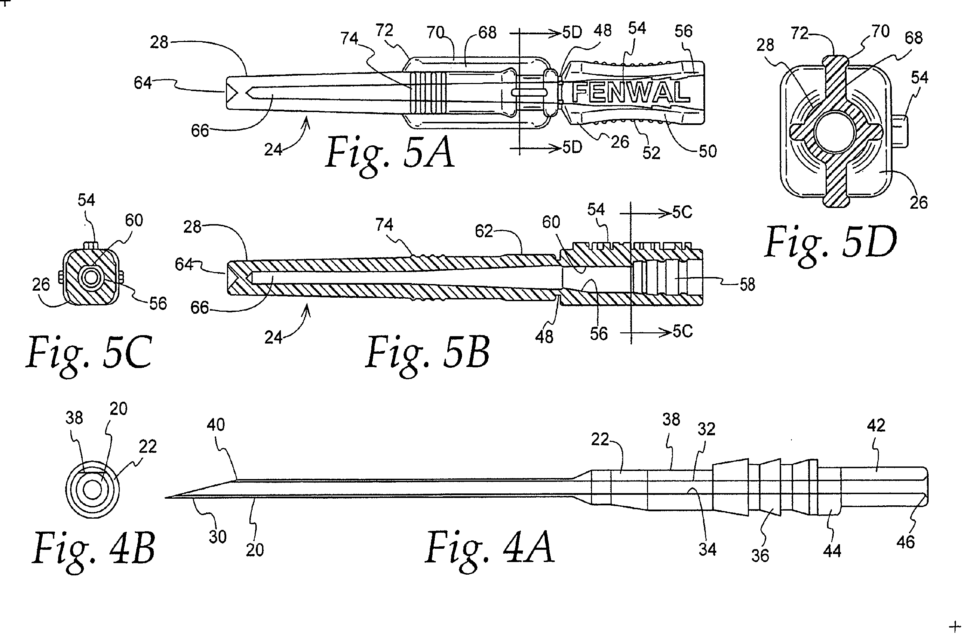

As shown in Figs. 3 and 4A, the illustrated base 22 includes a plurality of generally frusto-conical, radially extending rings or barbs 36, which interact with the needle cover 24 to secure the base 22 to the needle cover 24, as will be described in greater detail herein. It will be seen in Figs. 3-4B that the region of the base distal of the barbs 36 includes a flat portion 38. In the illustrated configuration, the base 22 is secured to the cannula 20 such that the flat portion 38 is aligned with the relatively blunt heel 40 of the beveled end 30 of the cannula 20. By such a configuration, the flat portion 38 effectively indicates the orientation of the beveled end 30, which is important for optimal phlebotomy of a donor. As will be described in greater detail herein, the flat portion 38 also serves the further function of ensuring a particular orientation of the beveled end 30 with respect to the needle cover 24 to optimize phlebotomy of a donor.

The proximal end 42 of the base 22 is adapted to form a liquid-tight connection with tubing 14 for association of the medical needle assembly 12 with a blood collection container or set. The base 22 may include a radial flange or abutment 44 against which the tubing 14 abuts when properly connected, thereby providing a visual indication that the tubing 14 is fully installed. In the illustrated embodiment, as best seen in Fig. 2C, the interior surface 46 of the proximal end 42 is tapered or beveled outwardly to smooth the transition from the cannula 20 to the tubing 14, thereby minimizing the occurrence of "dead zones" that would otherwise allow blood to collect and stagnate.

As for the needle cover 24 (Figs. 5A-5D), it comprises a proximal hub portion 26 and a distal cap portion 28. In the illustrated embodiments, the hub and cap portions are integrally attached, such as one-piece molded construction, and there is a frangible portion 48 proximal the cap portion 28, being more particularly positioned between the hub portion 26 and the cap portion 28. As will be described in greater detail, the frangible portion 48 is adapted to be broken by the phlebotomist, thereby allowing the cap portion 28 to be separated from the remainder of the needle cover 24 and removed, exposing the distal end of the cannula.

The hub portion 26 is intended to be gripped during use of the medical needle assembly 24, so it may be advantageous for it to be compact and easily manipulated between the fingertips of the phlebotomist. For example, the illustrated hub portions include concave sidewalls 50 which allow for comfortable gripping with the thumb and forefinger. The sidewalls 50 may further include gripping members 52 on the surfaces thereof. The gripping members 52 may be molded or embossed projections on the surfaces of the sidewalls 50 that affect the contour thereof. In the illustrated embodiments, the gripping members 52 are configured as spaced apart raised ribs on the sidewalls 50. An enhanced gripping surface can also be provided by other means, such as by treating the sidewalls to make them coarser or less smooth.

Other hub portion configurations may also be employed without departing from the scope of the present subject matter. For example, U.S. Patent Application Publication No. 2006/0089599, which is hereby incorporated herein by reference, shows and describes a number of alternative hub portion configurations.

In one embodiment, which is shown in Figs. 5A-5C, the outer surface of the hub portion 26 include an indicia 54, which extends generally longitudinally along the length of the hub portion 26. The indicia 54 may take any of a number of forms, but is distinguishable from the rest of the hub portion 26 to function as a reference point for the proper alignment of the beveled end 30 of cannula 20 during assembly and use, as will be described in greater detail herein.

The hub portion 26 includes an inner bore 56 (Figs. 2C and 5B) for receiving the base 22, which is itself secured to the cannula 20. The inner bore 56 is configured to substantially correspond to the shape of the outer surface of the base 22, such that there is a relatively tight fit when the base 22 is pressed into the bore 56 (Fig. 2C). In particular, the illustrated inner bore 56 includes annular notches, grooves, or recesses 58 corresponding to the barbs 36 of the base 22 and a flat portion 60 (shown in Figs. 5B and 5C) corresponding to the flat portion 38 of the base 22. When the base 22 is inserted into the hub portion 26 (moving the combined cannula 20 and base 22 from right-to-left in the orientation of Fig. 3), the flat portions 38 and 60 must be aligned to allow the base 22 to be fully inserted. With the flat portions 38 and 60 so aligned, the base 22 can be fully inserted into the inner bore 56 of the hub portion 26, with the barbs 36 seating within the associated annular notches 58, thereby preventing subsequent removal of the base 22 and cannula 20 from the hub portion 26.

As described above, the respective flat portions 38 and 60 of the base 22 and the hub portion 26 must be aligned for the base 22 to be fully inserted within the inner bore 56 of the hub portion 26. In the illustrated embodiments, the flat portion 60 of the hub portion 26 is aligned with the indicia 54 on the outer surface of the hub portion 26, while the flat portion 38 of the base 22 is aligned with the heel 40 of the beveled end 30 of the cannula 20. Therefore, alignment of the flat portions 38 and 60 effectively aligns the indicia 54 and the heel 40. It will be appreciated that, by such a configuration, the indicia 54 acts as a bevel indicator and a phlebotomist can ascertain the orientation of the beveled end 30 of the cannula 20 by simply referring to the position of the indicia 54 on the hub portion 26.

Turning now to the cap portion 28, the illustrated embodiment is tapered as it extends from an open proximal end 62 adjacent to the hub portion 26 to a closed distal end 64. An inner cavity 66 of the cap portion 28 (Figs. 2C and 5B) is sized to receive and substantially enclose at least the beveled distal end 30 and preferably the entire exposed length of the cannula 20 when the base 22 has been fully inserted into the inner bore 56 of the hub portion 26. In the embodiment illustrated in Fig. 2C, there is preferably spacing or separation between the surface of the inner cavity 66 and the cannula 20, which may be advantageous in preventing the sharp tip 30 of the cannula 20 from contacting and damaging the cap portion 28 during assembly.

The outer surface of the cap portion 28 may include at least one outwardly extending wing 68. The illustrated cap portion 28 defines a central longitudinal axis and a pair of wings 68 extending generally radially away from the central axis. In one embodiment, the wings 68 extend away from the central axis in generally opposite directions, such that they are substantially coplanar. Rising above the surface of each wing 68 is an enlarged rib 70 (Figs. 5A and 5D) that is adjacent to and/or defines at least a portion of an outer edge 72 of the associated wing 68. The illustrated enlarged ribs 70 are substantially parallel to the central axis of the cap portion 28. As will be described in greater detail, the cap portion 28 may be separated from the remainder of the needle cover 24 by application of a torsional force, and enlarged ribs according to the illustrated configuration may be advantageous for application of such torsional force. The cross-sectional surface (Fig. 5D) of each enlarged rib 70 may be curved or otherwise configured to provide a surface that is more comfortably gripped by the phlebotomist during application of such torsional force.

The cap portion 28 may also include at least one annular raised rib 74 that is positioned in a plane substantially perpendicular to the central axis. In the illustrated embodiments, the cap portion 28 includes a plurality of annular ribs 74 that are longitudinally spaced from each other. As will be described in greater detail herein, after breaking of the frangible portion 48, the cap portion 28 is moved distally to separate it from the remainder of the needle cover 24, thereby exposing the beveled distal end 30 of the cannula 20, and the annular ribs 74 function to provide a gripping surface when so removing the cap portion 28.

As for the frangible portion 48, it may be provided as a relatively weak section proximal the cap portion 28. In the illustrated embodiments, the frangible portion 48 is provided as a necked-down section of relative thin thickness intermediate the cap portion 28 and the hub portion 26. In accordance with the foregoing description, the cap portion 28 is adapted to be separated and removed from the remainder of the needle cover 24, and the frangible portion 48 is broken to allow the cap portion 28 to be so removed.

In one embodiment, the frangible portion 48 is adapted to break upon application of a torsional force. Such force is typically applied by gripping the hub portion 26 with one hand, gripping the cap portion 28 with another hand, and rotating the hub portion 26 and the cap portion 28 in opposite directions about the central axis defined by the cap portion 28. As described above, the wings 68 and (in particular) the enlarged ribs 70 are configured to be gripped during application of such torsional force, and it will be appreciated that positioning each enlarged rib 70 at the outer edge 72 of the associated wing 68 provides for a relatively large moment arm, thereby decreasing the force that must be applied by the phlebotomist to break the frangible portion 48. To minimize user discomfort, it may be further advantageous to provide a frangible portion 48 that is adapted to break upon application of a torsional force in the range from about 2 to about 24 in-ounces and upon less than about 90° relative rotation between the hub portion 26 and the cap portion 28. Additionally, as shown in Fig. 2C, it may be advantageous for there to be a slight friction fit between the distal end of the base 22 and the inner surface of the frangible portion 48 or the proximal end 62 of the cap portion 28 to prevent the cap portion 28 from falling off when the frangible portion 48 is broken. If provided, the raised annular ribs 74 on the cap portion 28 may be gripped to pull the cap portion 28 and overcome such friction fit.

The hub portion 26, cap portion 28, and frangible portion 48 may be integrally formed or, as in one embodiment, molded as a single piece. If so configured, it may be advantageous to construct the needle cover 24 from a material that: (1) is sufficiently rigid to minimize the bending of the underlying cannula 20, (2) is suitable for allowing the frangible portion 48 to be broken under the aforementioned conditions, and/or (3) has a moisture vapor transmission rate sufficient for steam sterilization of the beveled distal end 30 of the cannula 20 when it is enclosed by the cap portion 28 (e.g., a moisture vapor transmission rate in the range from about 30 to about 90 g/m²/day at approximately 38° C at 100% relative humidity). More particularly, it may be advantageous to make the hub, cap, and frangible portions of a plastic material, such as a material having a hardness in the range from about 35 to about 65 on the Shore D durometer scale, a torsional modulus in the range from about 30 to about 80 MPa, and/or a Young's modulus in the range from about 15 to about 260 MPa. Even more particularly, it may be advantageous to provide a plastic material having a hardness less than 55 on the Shore D durometer scale. More particularly still, it may be advantageous to provide a plastic material having a hardness of about 47 on the Shore D durometer scale.

An additional property that may be advantageous for the needle cover material to possess is translucence or, even more advantageously, substantial transparency. Such a material allows the beveled distal end of the cannula to be seen through the cap portion and allows the base to be seen through the hub portion (Figs. 2A and 2B). This allows the cannula and base to be visually inspected following assembly to assure that there has been no damage thereto during assembly.

One plastic material that has been found to possess these advantageous characteristics is the polyamide material marketed under the name VESTAMID® E47-S1 by Evonik Degussa GmbH of Essen, Germany. However, those of ordinary skill in the art will recognize that other materials may also be used, particularly if performance characteristics different from those recited herein are desired.

In other embodiments, the various sections of the needle protector may be comprised of different materials. For example, the hub portion may be formed from a softer material than the cap portion to provide different tactile and handling characteristics. Further, the construction of any portion of the needle protector is not limited to a single material. For example, the hub portion or the cap portion could include one or more sections formed from a material having a particular rigidity or translucence or transparency and one or more other sections formed from another material having different characteristics.

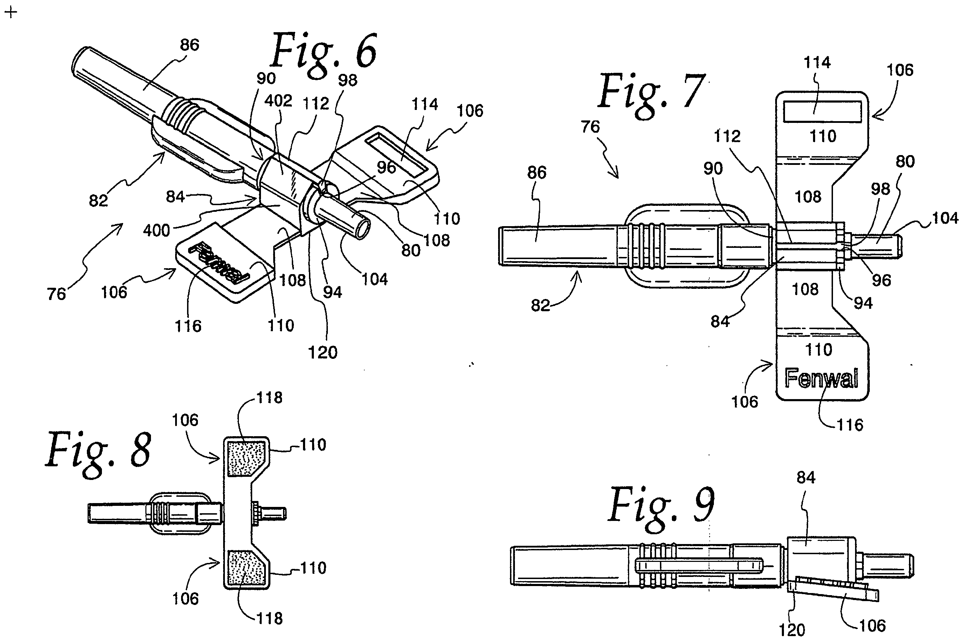

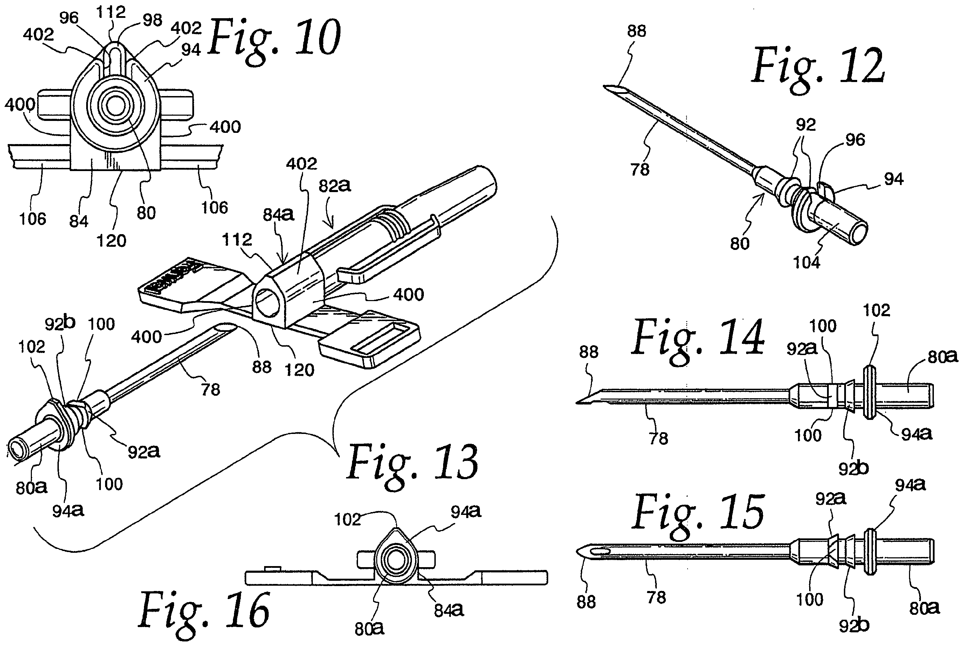

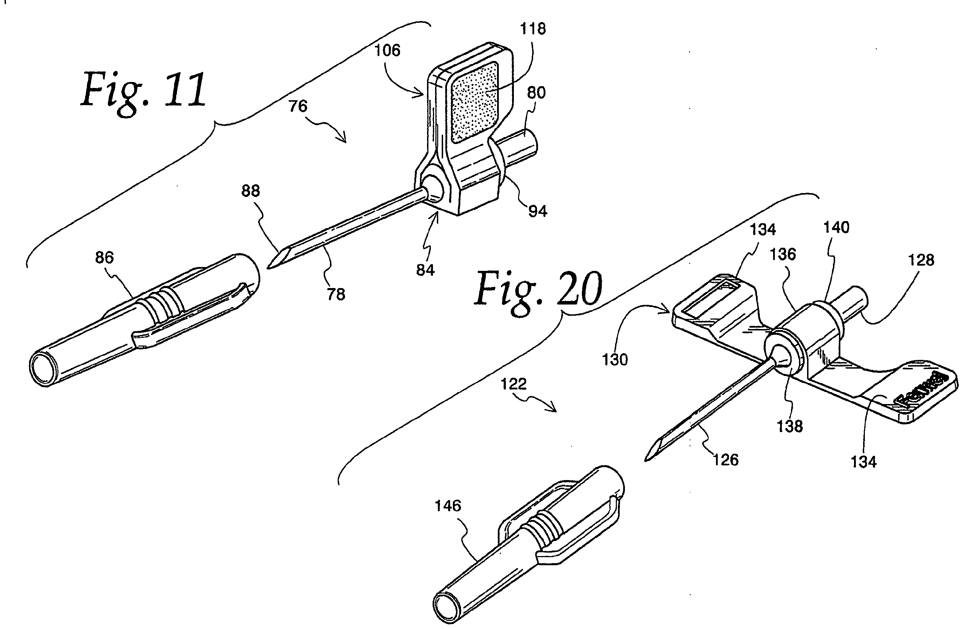

Figs. 6-11 show another medical needle assembly 76 embodying various aspects of the present subject matter. The assembly 76 includes a cannula or needle 78 (Fig. 12), a base or post 80 secured to the cannula 78, and a needle cover 82. The cannula 78 is secured to the base 80, and the base 80 is received within a hub portion 84 of the needle cover 82. A cap portion 86 of the needle cover 82 encloses at least a beveled distal end 88 (Fig. 12) of the cannula 78. The cap portion 86 is associated with the hub portion 84 by a frangible portion 90 that is broken to separate the cap portion 86 and uncover the cannula 78 (Fig. 11) for subsequent phlebotomization of a donor or patient. Except where otherwise noted, the components of the assembly 76 are provided in accordance with the foregoing description of the corresponding components of the embodiments of Figs. 2A-5D.

The base 80 may include retention members, such as interfering surfaces, adapted for securing it to a portion of the needle cover 82. In the embodiment illustrated in Fig. 12, the base 80 includes a plurality of generally frusto-conical, radially extending rings or barbs 92, which are received within notches of the hub portion 84 (not illustrated) to secure the base 80 to the needle cover 82, in accordance with the foregoing description of the embodiments of Figs. 2A-5D. Other means for securing the base 80 to the needle cover 82, such as an adhesive or the like, may also be employed without departing from the scope of the present disclosure.

To ensure a particular annular or angular orientation of the base 80 and cannula 78 within the needle cover 82, the base 80 and needle cover 82 may be provided with cooperating indexing surfaces or features. For example, in the embodiment of Figs. 6-11, the region of the base 80 proximal of the barbs 92 may be provided with an arcuate collar 94 having a channel 96 aligned with the beveled distal end 88 of the cannula 78. When the base 80 is pressed into the hub portion 84, the base 80 must be properly aligned so that a proximally extending peg or projection 98 of the hub portion 84 is received by the channel 96 (Figs. 6 and 10) to allow full insertion of the base 80. With the base 80 so oriented, the beveled distal end 88 of the cannula 78 will be aligned with the peg 98 of the hub portion 84, such that the peg 98 serves as a bevel indicator.

In another approach to orienting the cannula within the needle cover, illustrated in Figs. 13-16, at least one of the barbs of the base is provided with a flat portion. In the illustrated embodiment, the base 80a includes two frusto-conical barbs 92a and 92b, with the foremost barb 92a having top and bottom flat portions 100, one of which is aligned with the beveled distal end 88 of the cannula 78 (Figs. 14 and 15). An associated notch of the hub portion 84a has matching flat portions, thereby requiring the base 80a to be properly angularly oriented before it can be fully inserted into the needle cover 82a. A collar 94a proximal of the barbs 92a and 92b may be provided with a bevel indicator 102, illustrated as a pointed section, that is aligned with the beveled distal end 88 of the cannula 78. The collar 94a remains outside of the hub portion 84a (Fig. 16), with the bevel indicator 102 providing a visual indication that the assembly has been properly assembled, with the beveled distal end 88 of the cannula 78 properly oriented within the needle cover 82a. Other methods of orienting the cannula within the needle cover may also be employed, including the means described previously with regard to the embodiments of Figs. 2A-5D.

The proximal end 104 of the base 80/80a is preferably adapted to form a liquid-tight connection with tubing (not illustrated) for association of the medical needle assembly 82/82a with a blood collection container or set, as described above for the embodiments of Figs. 2A-5D.

As for the needle cover 80 (Figs. 6-11), the illustrated embodiment is provided generally in accordance with the foregoing description of the embodiments of Figs. 2A-5D, with the principal exceptions of a proximally extending peg 98 (described above) and a pair of flexible or foldable wings 106. The foldable wings 106 are adapted to be gripped during use of the medical needle assembly 76 and enhance the ease of handling during phlebotomization, while also providing a means for securing the needle assembly 76 to the skin of the patient or donor following phlebotomization, as will be described in greater detail herein.

Each illustrated foldable wing 106 may include a line of weakness such as a relatively thin and flexible section 108 extending outwardly from the hub portion 84 to a thicker grasping portion 110 to allow bending or folding of the foldable wing 106. The aforementioned polyamide material is sufficiently flexible to allow the foldable wings 106 to bend at the flexible sections 108 and press the grasping portions 110 against each other (Fig. 11), in a manner well known to those of skill in the art.

To assist in grasping and rotationally orienting the beveled distal end 88 of the cannula 78, the hub portion 84 may be provided with a configuration shape that the foldable wings 106 may contact when folded. For example, as shown in Fig. 6, the hub portion 84 may have a cross-sectional "steeple" configuration, with the top surface of the hub portion forming a peak or bevel indicator 112 that the flexible sections 108 overlay to guide the grasping portions 110 into contact with each other (Fig. 11). It will be seen in Fig. 10 that the hub portion 84 is, from an end or cross-sectional view, generally pentagonal or "home plate"-shaped, with a flat bottom surface 120, two upwardly extending sidewalls 400, and two inclined surfaces or guidewalls 402 which meet or intersect at the bevel indicator 112. This arrangement provides an upwardly extending orientation member or non-circular wing-guiding member that may be closely contacted by the surfaces of the wings 106, when folded, to allow greater gripping forces to be exerted on the hub portion 84 by the user when gripping the wings 106 in the folded position.

The illustrated sidewalls 400 are generally perpendicular to the bottom surface 120, generally planar, and generally parallel to each other. At the upper ends of the sidewalls 400, there is an angular transition between the sidewalls 400 and the guidewalls 402. The transition between the sidewalls 400 and the guidewalls 402 may be either continuous (i.e., smoothly curved) or more pronounced (e.g., forming a defined edge or angle therebetween). The guidewalls 402 are angled toward each other to meet at the bevel indicator 112. In the illustrated embodiment, the guidewalls 402 are substantially planar to present the illustrated pentagonal or "home plate"-shaped cross-section, but other configurations are also within the scope of the present disclosure, such as guidewalls that are concave or convex from an end view.

The intersection of the guidewalls 402 at the bevel indicator 112 may be either continuous (i.e., curved) or discontinuous (e.g., forming a defined edge or angle or flattened section therebetween), thereby allowing for a variety of different bevel indicator configurations. For example, Fig. 10 shows a bevel indicator 112 that is generally curved, while still being clearly defined between the guidewalls 402, and Fig. 13 shows a bevel indicator 112 that is flattened and generally parallel to the bottom surface 120 of the hub portion 84a. It is advantageous for the bevel indicator 112 to be discernible from the guidewalls 402, rather than defining a uniform circular arc with the guidewalls 402, so as to serve as a visual bevel indicator to the user.

The "steeple" configuration is merely illustrative and other hub portion configurations including a raised orientation member or non-circular wing-guiding member may be employed without departing from the scope of the present disclosure. Benefits of a hub portion with a raised orientation member or non-circular wing-guiding member include providing a more rigid surface than the foldable wings 106 when applying the torque required to break the frangible portion 90. The ability to press the wings 106 against the orientation member provides a more solid feel for the user when attempting to grasp the wings 106 and properly position the beveled distal end 88 of the cannula 78 prior to venipuncture. Further, the raised orientation member provides an additional contact point on the hub portion 84 during manufacturing when the cannula 78 and base 80 are pressed into the needle cover 82.

The grasping portions 110 may be provided with a slide resistance or interlock features to prevent the grasping portions 110 from sliding relative to each other when pressed together. In the illustrated embodiment, one interlock feature (indicated at 114) is generally concave and the other interlock feature (indicated at 116) is generally convex to fit together when the grasping portions 110 are pressed together for manipulation of the needle assembly 76 during phlebotomization. With the grasping portions 110 pressed together (Fig. 11), the phlebotomist locates and pierces a vein with the beveled distal end 88 of the cannula 78. A needle assembly including foldable wings may be considered advantageous by some users compared to one lacking such features, as the foldable wings keep the fingers of the phlebotomist away from the hub portion during phlebotomization, thereby allowing for a shallower entry angle into the target vein.

To provide additional traction for the fingers of the phlebotomist, the underside of one or both of the grasping portions 110 may be provided with anti-slip features. For example, in the embodiment of Figs. 8 and 11, the underside of the grasping portions 110 include grip pads 118 that are knurled or otherwise textured to provide additional friction to reduce slippage during phlebotomization.

When the beveled distal end 88 of the cannula 78 is properly positioned within the vein, the foldable wings 106 may be released, allowing them to return substantially to their original configuration due to material memory. Thereafter, the foldable wings 106 may be secured to the skin, e.g., the arm of the patient/donor, in a manner well known to those of skill in the art.

In addition to the foregoing, the hub portion 84 may be provided with a bottom surface 120 that is angled with respect to the central axis of the cannula 78 (Fig. 9), rather than being substantially parallel thereto. The bottom surface 120 may be angled to generally match the typical entry angle of the cannula tip 88 into a vein, thereby limiting interfering contact with the patient's skin, making it easier to insert the cannula tip 88 into the vein, and reducing the stress on the cannula 78 and the vein entry site when the foldable wings 106 are secured to the arm of the donor or patient.

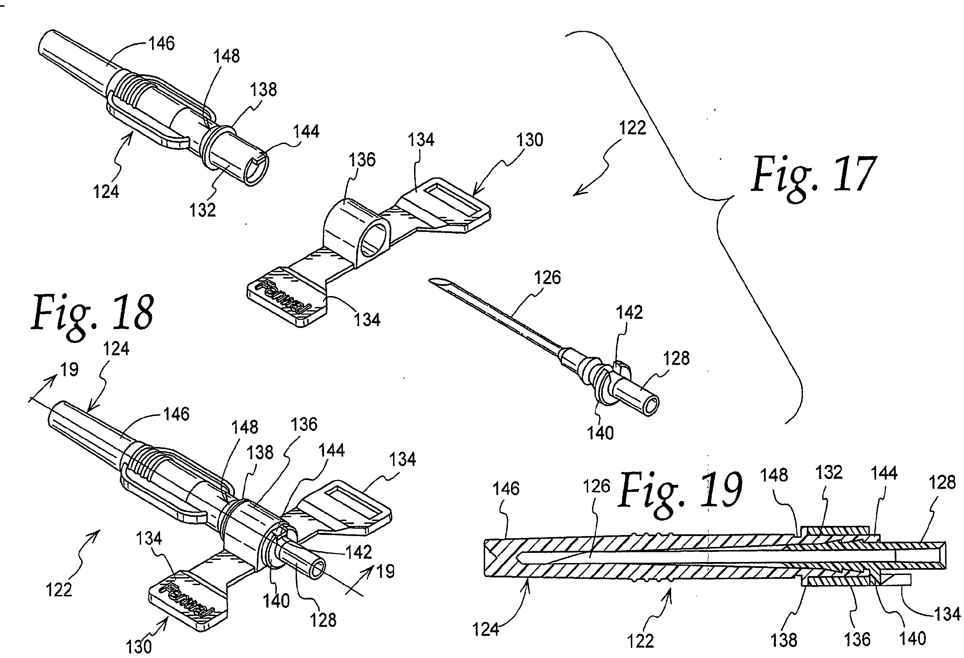

In another embodiment, illustrated in Figs. 17-20, instead of being integrally formed with the hub portion, the foldable wings may be a separate component, as illustrated in Fig. 17, whereby a needle assembly 122 comprises a needle cover 124, a cannula 126 and base 128, and a separate winged attachment piece 130.

In accordance with the embodiments described previously, the cannula 126 and associated base 128 are inserted into the needle cover 124, but in the embodiment of Figs. 17-20, the winged attachment piece 130 is separate and placed onto the hub portion 132 of the needle cover 124 prior to securing the base 128 to the hub portion 132. More particularly, the illustrated winged attachment piece 130 has a pair of foldable wings 134, corresponding to the foregoing description of the foldable wings of the embodiment of Figs. 6-11, meeting at a central ring portion 136. The ring portion 136 has an inner diameter that is slightly greater than the outer diameter of the hub portion 132, allowing the ring portion 136 to be seated around the hub portion 132 (Figs. 18 and 19).

The hub portion 132 and/or the base 128 may be provided with features adapted to prevent or restrict free relative movement of the winged attachment piece 130. For example, in the embodiment of Fig. 17, the hub portion 132 includes an outwardly extending annular lip 138 adapted to abut the front end of the ring portion 136 when the ring portion 136 is seated on the hub portion 132. Similarly, a collar 140 of the base 128 may provide a surface that abuts the rear end of the ring portion 136 when the needle assembly 122 is fully assembled (Figs. 18 and 19). The illustrated collar 140 is substantially identical to the collar incorporated in the base of Fig. 12 and includes a channel 142 adapted to receive a proximally extending projection or peg 144 of the hub portion 132 to properly orient the cannula 126 within the needle cover 124, in accordance with the foregoing description of the embodiment of Figs. 6-11.

Together, the lip 138 and the collar 140 prevent the winged attachment piece 130 from sliding longitudinally with respect to the rest of the needle assembly, while allowing the winged attachment piece 130 to rotate with respect to the rest of the needle assembly. Rotation of the winged attachment piece 130, and the foldable wings 134 in particular, may be advantageous after the foldable wings 134 have been secured to the arm of a donor or patient. If blood flow from the vein slows, for example due to the beveled distal end of the cannula being pressed against the vein wall, the cannula may be rotated with respect to the foldable wings to improve blood flow out of the vein. In this manner, the foldable wings 134 may remain secured or taped to the patient's skin and the medical practitioner can still adjust the position of the needle bevel.

On the other hand, while it may be advantageous for the winged attachment piece 130 to be rotatable with respect to the rest of the needle assembly after the foldable wings 134 have been secured to a donor or patient, it may be disadvantageous for them to rotate prior to the initial phlebotomization. If the frangible portion 148 of the needle cover 124 is adapted to break upon application of torque thereto for removing the cap portion 146 and exposing the tip of the cannula 126 (Fig. 20), a freely rotatable winged attachment piece (which overlays the hub portion 132) may hinder application of the necessary torque, meaning that the frangible portion 148 cannot be broken to expose the beveled distal end of the cannula 126. To avoid such free rotation prior to phlebotomization, a temporary or conditional anti-rotation feature may be incorporated into the needle assembly. For example, interfitting teeth, such as a ratchet, may be employed between the ring portion 136 and the hub portion 132 which allows rotation in a particular direction (to allow for adjustment of the cannula when the foldable wings are secured to the arm of a donor or patient), while preventing rotation in the opposite direction to allow the phlebotomist to break the frangible portion 148. Other temporary or anti-rotation features may also be employed without departing from the scope of the present disclosure.

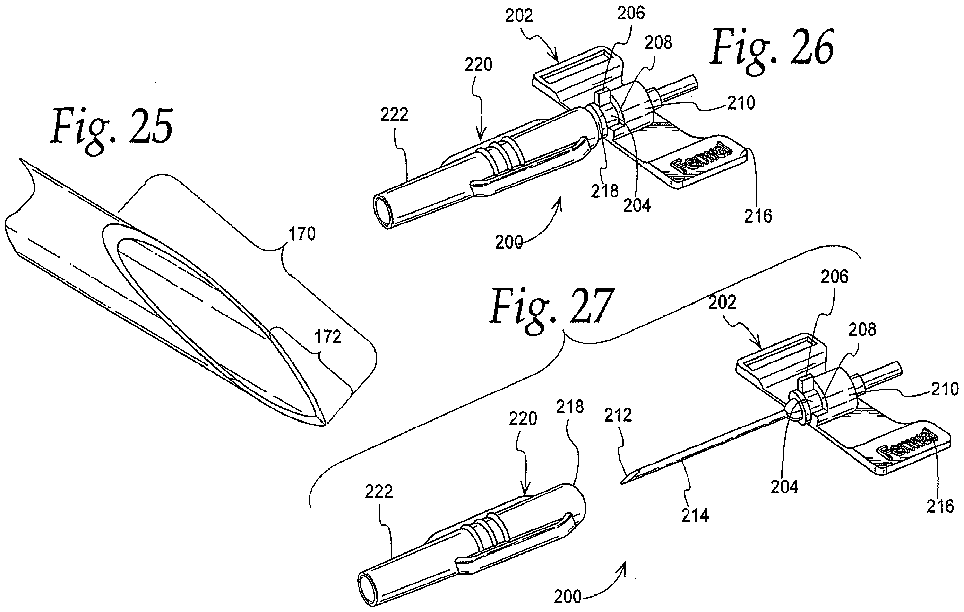

For example, Figs. 26-29 illustrate a needle assembly 200 incorporating a winged attachment piece 202 that is selectively rotatable with respect to the underlying hub portion 204. The foregoing description of the embodiment illustrated in Figs. 17-20 is generally applicable to this assembly, except as noted below.

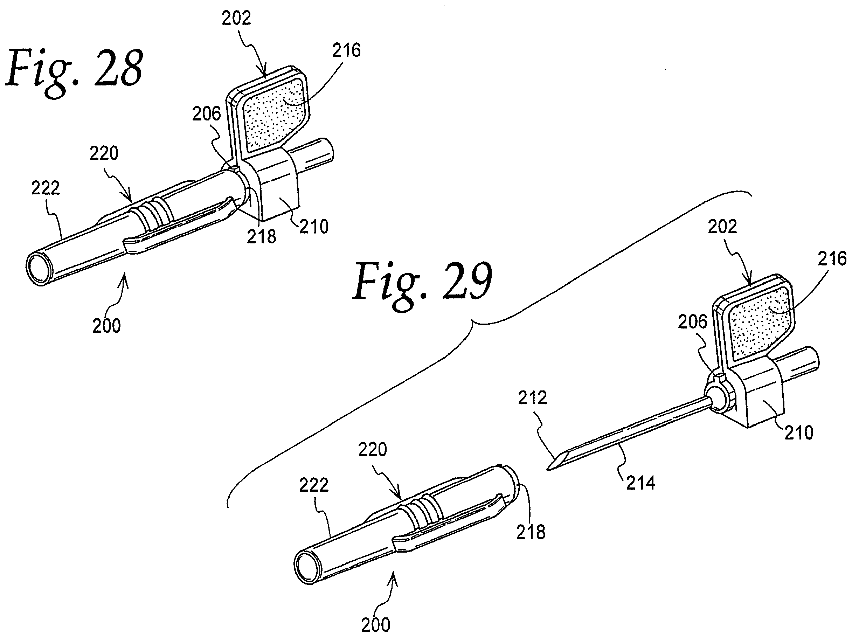

The needle assembly 200 of Figs. 26-29 is provided with a hub portion 204 including a hub anti-rotation member 206 that is adapted for selective anti-rotational engagement with a portion of the winged attachment piece 202, as will be described in greater detail herein. In the illustrated embodiment, the hub anti-rotation member 206 is provided as a hub projection that extends radially outwardly and is configured to be selectively pinched between the wings of the winged attachment piece 202, as shown in Figs. 28 and 29 and as will be described in greater detail herein. However, the hub anti-rotation member is not limited to an outward hub projection suited to be pinched between the wings of the winged attachment piece, but instead encompasses any hub feature that is engageable with any portion of the winged attachment piece to selectively prevent relative rotation between the hub portion and the winged attachment piece.

In the embodiment of Figs. 26-29, the hub anti-rotation member 206 is a radial projection from the hub portion 204 at least partially received within a groove 208 formed in the central ring portion 210 of the winged attachment piece 202. The hub anti-rotation member 206 and the groove 208, if provided, may be variously configured and positioned without departing from the scope of the present disclosure, provided that there is an interfitting movable relationship that allows for at least limited relative rotation between the hub portion 204 and the winged attachment piece 202. For example, the illustrated embodiment employs a hub anti-rotation member 206 and groove 208 positioned at distal sections of the hub portion 204 and the central ring portion 210, respectively, which allows the hub anti-rotation member 206 to slide into the groove 208 during assembly. A similar effect may be achieved by providing the hub anti-rotation member 206 and the groove 208 at proximal sections of the hub portion 204 and the central ring portion 210, respectively. It may be further advantageous for the hub anti-rotation member 206 to be aligned with the beveled distal end 212 of the cannula 214 (Fig. 27) to serve as an orientation feature that indicates the location of the beveled distal end 212 while the cannula 214 is in place within a vein.

The winged attachment piece 202 is rotatable about the hub portion 204, with the hub anti-rotation member 206 moving within the groove 208 (if provided) until it abuts an end of the groove 208. Accordingly, it may be advantageous for the groove 208 to be substantially wider than the hub anti-rotation member 206 to allow a greater degree of rotation once the foldable wings 216 have been secured to the donor or patient. However, as described previously, it may be disadvantageous for the foldable wings 216 to rotate prior to the initial phlebotomization, particularly when the user is attempting to apply sufficient torque so as to break the frangible portion 218 of the needle cover 220 and expose the tip of the cannula 214. To prevent such free rotation prior to phlebotomization, the hub anti-rotation member 206 selectively engages a portion of the winged attachment piece 202 in a rotationally fixed manner. In the illustrated embodiment, this is achieved by pinching the foldable wings 216 of the winged attachment piece 202 together to trap the hub anti-rotation member 206 therebetween (Fig. 28). So trapping the hub anti-rotation member 206 between the foldable wings 216 prevents relative rotation between the winged attachment piece 202 and the remainder of the needle assembly 200, thereby holding the winged attachment piece 202 in place while the cap portion 222 of the needle cover 220 is gripped with the other hand and twisted for removal (Fig. 29).

When the cap portion 222 has been broken away from the remainder of the needle assembly 200, the donor or patient is phlebotomized and the wings 216 may be released and subsequently secured to the body. With the wings 216 so released, the hub portion 204 is again free to rotate with respect to the winged attachment piece 202, thereby allowing the phlebotomist to adjust the location of the bevel end 212 of the cannula 214 within the vein.

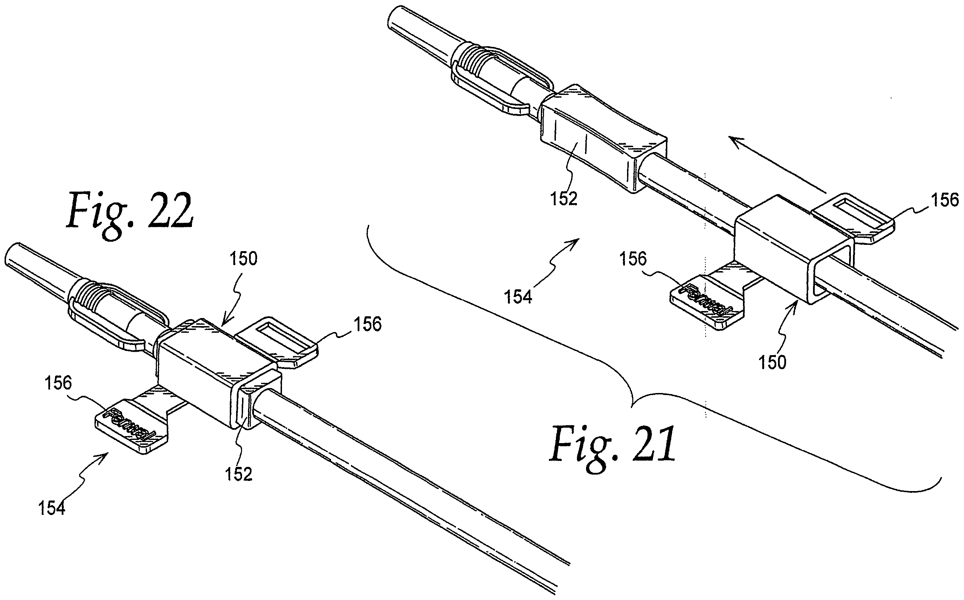

The embodiment of Figs. 17-20 is provided with a winged attachment piece 130 that is separate from the rest of the assembly, but is not configured to be removed by the user. Figs. 21 and 22 illustrate a variation of the embodiment of Figs. 17-20, wherein an alternative winged attachment piece 150 is provided that may be attached and/or removed at the user's discretion. For example, a needle assembly with a wingless hub portion 152, such as the embodiment illustrated in Figs. 2A-5D, may be provided, along with a separate winged attachment piece 150 (Fig. 21). The winged attachment piece 150 may be initially associated with the hub portion 152 (Fig. 22), resulting in a needle assembly 154 comparable to the embodiment illustrated in Figs. 17-20. If the user does not wish to use the foldable wings 156, the winged attachment piece 150 may be removed from the hub portion 152. The manner of removing the winged attachment piece 150 will vary depending on the nature of the association between the winged attachment piece 150 and the hub portion 152. In one embodiment, the winged attachment piece 150 is snap-fit onto the hub portion 152, so it may be removed by an unsnapping operation. In another embodiment, the winged attachment piece 150 is slid onto the hub portion 152, so it may be removed by sliding the winged attachment piece 150 off of the hub portion 152. Once the winged attachment piece 150 is removed, it can later be attached as need be.

Alternatively, rather than having the winged attachment piece 150 initially associated with the hub portion 152, it may be initially provided separate therefrom (Fig. 21), to be later attached by the user (Fig. 22) if so desired. Any of a number of attachment methods may be employed such as, but not limited to, a snap-on or a slide-on operation. Once the winged attachment piece 150 is attached to the hub portion 152, it can later be removed as need be.

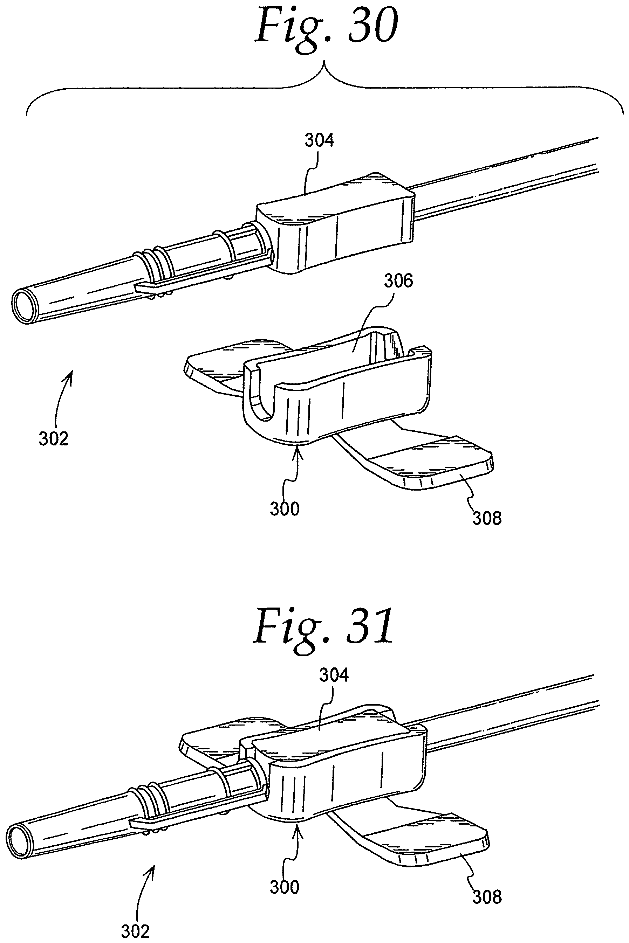

Figs. 30 and 31 illustrate another embodiment of a winged attachment piece 300 that is separable from the rest of the needle assembly 302. For example, a needle assembly 302 with a wingless hub portion 304, such as the embodiment illustrated in Figs. 2A-5D, may be provided, along with a separate winged attachment piece 300 (Fig. 30).

The illustrated winged attachment piece 300 has a centrally positioned cavity 306 (Fig. 30) adapted to receive the hub portion 304 of the needle assembly 302 (Fig. 31), allowing the winged attachment piece 300 to be snap-fit onto the hub portion 304 if the user prefers a needle assembly with foldable wings 308. Later, the winged attachment piece 300 may be removed by an unsnapping operation. Once the winged attachment piece 300 is removed, it can subsequently be attached as need be.

Alternatively, rather than having the winged attachment piece 300 initially separate from the hub portion 304, it may be initially connected thereto (Fig. 31), to be removed by the user (Fig. 30) if so desired.

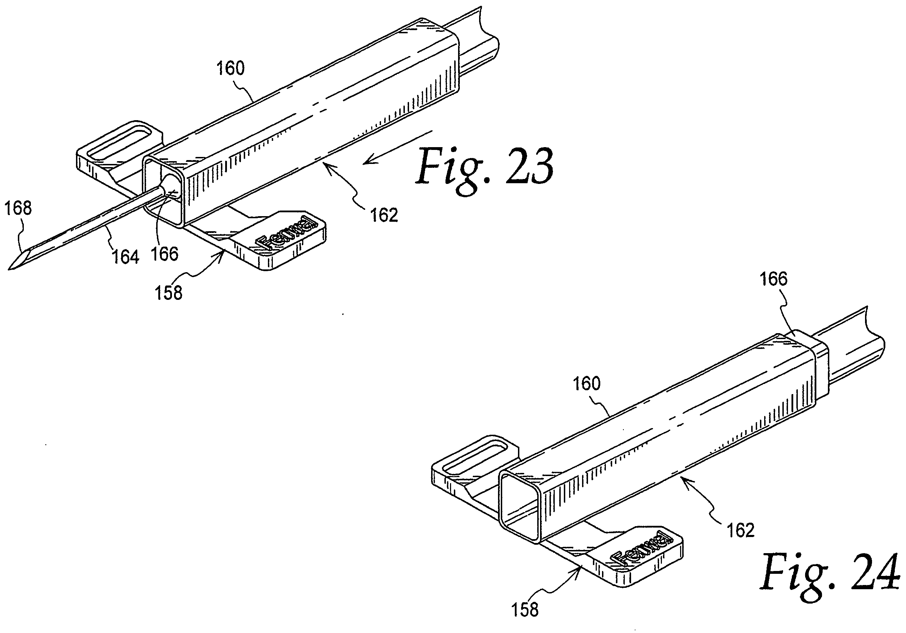

In yet another embodiment, illustrated in Figs. 23 and 24, the winged attachment piece may be adapted to serve as a needle shield after the cannula is removed from a vein. In such an embodiment, the winged attachment piece 158 or a portion thereof (e.g., the central ring portion 160) may be relatively elongated, compared to the embodiment of Figs. 17-20. Before and during phlebotomy, the needle assembly 162 and winged attachment piece 158 are in the condition illustrated in Fig. 23 and operate according to the foregoing description of the embodiment of Figs. 17-20. However, when the cannula 164 is removed from the vein, the winged attachment piece 158 is moved distally along the hub portion 166 until it (particularly the central ring portion 160) at least partially encloses the cannula 164, most preferably moving far enough to enclose the beveled distal end 168 of the cannula 164 (Fig. 24). This may be a sliding movement, but is not limited to any particular manner of relative axial advancement.

As described above, the winged attachment piece 158 is relatively elongated, the reason being so that it may enclose a portion of the cannula 164 while remaining at least partially associated with the hub portion 166. To ensure that the winged attachment piece 158 remains in the shielding condition of Fig. 24, the winged attachment piece 158 and/or the hub portion 166 may be provided with interfering surfaces that prevent proximal movement of the winged attachment piece 158. With the winged attachment piece 158 so locked in place, it serves as a needle shield that prevents inadvertent needle stick following phlebotomization.

Medical needle assemblies according to the foregoing description have particular applicability to blood collection and processing systems. For example, it is contemplated that such a medical needle assembly could be incorporated into any of the various BLOOD-PACK® Units marketed by Fenwal, Inc. of Lake Zurich, Illinois. However, needle assemblies according to the foregoing description are not limited to a particular use or range of uses, a particular needle gauge or configuration (e.g., the present disclosure may be employed with a cannula having a back-eye) and it is contemplated that such medical needle assemblies could be incorporated in any system and assembly involving needle entry into the vascular system of a patient or donor.

One obstacle to ready incorporation of needle protectors according to the present disclosure into both manual systems and automated systems is the fact that they typically employ different needles. Automated blood extraction system typically employ a relatively small gauge fistula needle because blood is actively pumped or drawn from a donor or patient, as opposed to manual applications in which the rate of blood removal is dictated by the donor or patient's blood pressure and a larger gauge phlebotomy needle is employed. While this difference in needles may be accommodated by providing differently sized or configured needle protectors, a single needle protector may be employed with both types of needles if a larger gauge fistula needle (i.e., one having the same gauge as a typical phlebotomy needle) can be provided.

According to one aspect of the present disclosure, a larger gauge fistula needle is provided without sacrificing flow capacity. This may be achieved by various means, but in an exemplary embodiment, the flow capacity of the fistula needle is increased by reducing the flow resistance, specifically by decreasing the overall length of the needle. An illustrative example of such an improved fistula needle follows, but those having skill in the art will appreciate that the principle described herein may be employed with a phlebotomy or fistula needle of any gauge.

A beveled cannula tip having a primary bevel 170 and a secondary bevel 172 is generically illustrated in Fig. 25. Dual-bevel needles, such as those described in U.S. Patent Application Publication No. 2004/0082899, which is hereby incorporated herein by reference, are well-known to those of skill in the art. By way of example, one known system employs a 16-gauge phlebotomy needle having an overall length of approximately 2.695 inches, with an outside diameter of approximately 0.065 inch and an inside diameter of approximately 0.0565 inch. The primary bevel 170 has a length of approximately 0.245 inch and the secondary bevel 172 has a length of approximately 0.100 inch. Such a needle has a theoretical flow rate of approximately 64.45 ml/min and a measured flow rate of approximately 61.75 ml/min under selected pressure conditions. A known 17-gauge phlebotomy needle has an overall length of approximately 2.695 inches, with an outside diameter of approximately 0.058 inch and an inside diameter of approximately 0.050 inch. The primary bevel 170 has a length of approximately 0.209 inch and the secondary bevel 172 has a length of approximately 0.129 inch. Such a needle has a theoretical flow rate of approximately 50.72 ml/min and a measured flow rate of approximately 47.3 ml/min under comparable flow pressures. Hence, such a 17-gauge needle experiences an approximately 23.4% flow reduction as compared to the aforementioned 16-gauge needle.

To provide a 17-gauge fistula needle having similar flow capacity to the known 16-gauge needle, the overall length of the needle is reduced. For example, a 17-gauge needle may be provided with an overall length of approximately 1.665 inches, with an outside diameter of approximately 0.058 inch and an inside diameter of approximately 0.050 inch. The primary bevel 170 may have a length of approximately 0.186 inch and the secondary bevel 172 may have a length of approximately 0.072 inch. Such a needle has a theoretical flow rate of approximately 64.31 ml/min and a measured flow rate of approximately 60.5 ml/min, which is substantially the same as the aforementioned 16-gauge needle in comparable flow pressure conditions. Although the inside diameter of such a needle is smaller than the inside diameter of the known 16-gauge needle, the reduced length offsets the pressure drop to allow for similar flow capacity.

In additional to the functional advantages of this improved 17-gauge fistula needle, there are other advantages as well. First, such a needle has the same gauge as a known phlebotomy needle so, when compared to needles having different gauges, the two needles may be more easily employed with needle protectors according to the present disclosure. Second, such a needle decreases donor anxiety because it is shorter and has a smaller outside diameter. Additionally, donor discomfort is also decreased, because the smaller gauge results in a smaller vein puncture site.

## Claims

1. A medical needle assembly (76) comprising:
a cannula (78) including a beveled distal end (88);
a base (80) secured to the cannula (78) and including a channel (96); and
a needle cover (82) comprising a hub portion (84, 84a) adapted to be secured to the base (80), wherein the hub portion (84) includes
a bottom surface (120),
a pair of sidewalls (400) extending from the bottom surface (120),
a pair of guidewalls (402) extending toward each other from upper ends of the sidewalls (400) and intersecting at a bevel indicator (112),
a projection (98) aligned with the bevel indicator (112), extending proximally away from the bevel indicator (112), and configured to be received by the channel (96) when the hub portion (84) is secured to the base (80), and
a pair of wings (106) extending outwardly from said sidewalls (400), and wherein
the wings (106) are foldable to overlay at least a portion of the guidewalls (402) and contact the guidewalls (402) and each other, and
the beveled distal end (88) of the cannula (78) is configured to be aligned with the projection (98) and the channel (96) when the hub portion (84) is secured to the base (80).

2. The medical needle assembly (76) according to claim 1, wherein the bottom surface (120) is flat, and the sidewalls (400) are perpendicular to the bottom surface (120), planar, and parallel to each other.

3. The medical needle assembly (76) according to claim 1 or claim 2, wherein the guidewalls (402) are planar.

4. The medical needle assembly (76) according to any one of the preceding claims, wherein the cross-section of the hub portion (84) has a "steeple" configuration.

5. The medical needle assembly (76) according to any one of the preceding claims, wherein the cannula (78) has a central axis and the bottom surface (120) of the hub portion (84) is configured at an angle with respect to said central axis when the hub portion (84) is secured to the base (80).

## Patentansprüche

1. Medizinische Nadelanordnung (76), umfassend:
eine Kanüle (78), die ein abgeschrägtes distales Ende (88) enthält;
eine Basis (80), die an der Kanüle (78) befestigt ist und einen Kanal (96) enthält; und
eine Nadelabdeckung (82), die einen Nabenabschnitt (84, 84a) umfasst, der angepasst ist, an der Basis befestigt zu werden, wobei der Nabenabschnitt (84) enthält
eine Bodenfläche (120),
ein Paar Seitenwände (400), die sich von der Bodenfläche (120) erstrecken,
eine Paar Führungswände (402), die sich von oberen Enden der Seitenwände (400) zueinander hin erstrecken und sich an einem abgeschrägten Indikator (112) schneiden,
einen Vorsprung (98), der mit dem abgeschrägten Indikator (112) ausgerichtet ist, sich proximal weg von dem abgeschrägten Indikator (112) erstreckend, und konfiguriert ist, von dem Kanal (96) aufgenommen zu werden, wenn der Nabenabschnitt (84) an der Basis (80) befestigt wird, und
ein Paar Flügel (106), die sich von den Seitenwänden (400) nach außen erstrecken, und wobei
die Flügel (106) klappbar sind, um zumindest einen Abschnitt der Führungswände (402) zu überlagern und die Führungswände (402) und sich gegenseitig zu kontaktieren, und
das abgeschrägte distale Ende (88) der Kanüle (78) konfiguriert ist, mit dem Vorsprung (98) und dem Kanal (96) ausgerichtet zu werden, wenn der Nabenabschnitt (84) an der Basis (80) befestigt wird.

2. Medizinische Nadelanordnung (76) nach Anspruch 1, wobei die Bodenfläche (120) flach ist und die Seitenwände (400) senkrecht zu der Bodenfläche (120), planar, und parallel zueinander sind.

3. Medizinische Nadelanordnung (76) nach Anspruch 1 oder Anspruch 2, wobei die Führungswände (402) planar sind.

4. Medizinische Nadelanordnung (76) nach einem der vorhergehenden Ansprüche, wobei der Querschnitt des Nabenabschnitts (84) eine "Kirchturm-" (engl. "steeple") Konfiguration aufweist.

5. Medizinische Nadelanordnung (76) nach einem der vorhergehenden Ansprüche, wobei die Kanüle (78) eine zentrale Achse aufweist und die Bodenfläche (120) des Nabenabschnitts (84) in einem Winkel bezüglich der zentralen Achse konfiguriert ist, wenn der Nabenabschnitt (84) an der Basis (80) befestigt ist.

## Revendications

1. Ensemble d'aiguille médicale (76) comprenant :
une canule (78) comportant une extrémité distale biseautée (88) ;
une base (80) fixée sur la canule (78) et comportant un canal (96) ; et
une protection d'aiguille (82) comprenant une partie de moyeu (84, 84a) adaptée de manière à être fixée sur la base (80), dans lequel la partie de moyeu (84) comporte
une surface inférieure (120),
une paire de parois latérales (400) s'étendant à partir de la surface inférieure (120),
une paire de parois de guidage (402) s'étendant l'une vers l'autre à partir des extrémités supérieures des parois latérales (400) et se coupant au niveau d'un indicateur biseauté (112),
une saillie (98) alignée avec l'indicateur biseauté (112), s'écartant de manière proximale de l'indicateur biseauté (112) et configurée de manière à être reçue par le canal (96) lorsque la partie de moyeu (84) est fixée sur la base (80), et
une paire d'ailettes (106) s'étendant vers l'extérieur à partir desdites parois latérales (400), et dans lequel
les ailettes (106) peuvent être repliées afin de recouvrir au moins une partie des parois de guidage (402) et d'entrer en contact avec les parois de guidage (402), et l'une avec l'autre, et
l'extrémité distale biseautée (88) de la canule (78) est configurée de manière à être alignée avec la saillie (98) et le canal (96) lorsque la partie de moyeu (84) est fixée sur la base (80).

2. Ensemble d'aiguille médicale (76) selon la revendication 1, dans lequel la surface inférieure (120) est plate et les parois latérales (400) sont perpendiculaires à la surface inférieure (120), planes et parallèles l'une à l'autre.

3. Ensemble d'aiguille médicale (76) selon la revendication 1 ou 2, dans lequel les parois de guidage (402) sont planes.

4. Ensemble d'aiguille médicale (76) selon l'une quelconque des revendications précédentes, dans lequel la section transversale de la partie de moyeu (84) présente une configuration en "fléchette".

5. Ensemble d'aiguille médicale (76) selon l'une quelconque des revendications précédentes, dans lequel la canule (78) comporte un axe central et la surface inférieure (120) de la partie de moyeu (84) est configurée sous un certain angle par rapport audit axe central lorsque la partie de moyeu (84) est fixée sur la base (80).
